# EUROPÄISCHE PATENTSCHRIFT

(11) **EP 1 188 431 B1**
(45) Veröffentlichungstag und Bekanntmachung des Hinweises auf die Patenterteilung: **17.11.2004**
(21) Anmeldenummer: 01122272.6
(22) Anmeldetag: 18.09.2001
(51) Int. Cl.: A61K 7/09

(54) **Verfahren zum Dauerwellen von menschlichen Haaren**
Method for perming human hair
Procédé de deformation permanente des cheveux humains

(30) Priorität: 19.09.2000 DE 10046255
(43) Veröffentlichungstag der Anmeldung: 20.03.2002
(73) Patentinhaber: KPSS-Kao Professional Salon Services GmbH, 64297 Darmstadt (DE)
(72) Erfinder: Grit, Mustafa, Dr., 64579 Gernsheim (DE); Dubowoj, Polina, Aree Place Condominium Room 801, Klongton, Klongtoey, Bangkok 10110 (TH)

(56) Entgegenhaltungen:
- EP-A- 0 692 248
- DE-A- 2 822 125
- DE-A- 19 955 842
- DE-U- 29 817 970
- CHEMICAL ABSTRACTS, vol. 119, no. 8, 1993 Columbus, Ohio, US; abstract no. 79821a, HOYU KK: "Hair preparations for modifying curly hairs" Seite 488; Spalte 1; XP002221600 & JP 05 117134 A 14. Mai 1993 (1993-05-14)
- VALERIE PARISON: "Active delivery from Nylon particles" COSMETICS & TOILETRIES, WHEATON, IL, US, Bd. 108, Nr. 12, 1. Dezember 1993 (1993-12-01), Seiten 97-100, XP002085590 ISSN: 0361-4387

## Beschreibung

Die vorliegende Erfindung betrifft ein neues Verfahren zum dauerhaften Verformen, d.h. Dauerwellen, von menschlichen Haaren, durch das die unangenehme Geruchsentwicklung nach der Verwendung von Thiogruppen enthaltenden Reduktionsmitteln auf dauergewelltem Haar weitgehend beseitigt wird.

Es ist ein seit langem bekanntes, jedoch nach wie vor ungelöstes Problem, daß dauergewelltes Haar auch nach erfolgter Fixierung bzw. Neutralisation noch einen als unangenehm empfundenen Geruch nach dem als Verformungsmittel verwendeten Reduktionsmittel aufweist, obwohl das Haar nach der Fixierung üblicherweise mit Parfum enthaltenden Nachbehandlungsmitteln wie Shampoos, Spülungen, Kuren, etc. behandelt wird.

Es wurde auch schon vorgeschlagen, die Parfumierung von Dauerwellpräparaten und Fixiermitteln hierfür dadurch zu verbessern, daß das Parfum der entsprechenden Zusammensetzung erst unmittelbar vor der Anwendung zugesetzt wird (DE 36 40 748 C1).
Doch auch dadurch läßt sich das Problem nicht lösen.

Es wurde nun gefunden, und das ist Gegenstand der vorliegenden Erfindung, daß sich eine weitgehende und dauerhafte Entfernung des Reduktionsmittelgeruchs aus dem dauergewellten Haar dadurch erreichen läßt, wenn das verformte Haar nach dem Entfernen des Reduktionsmittels und/oder Fixiermittels das Haar mit einem Präparat behandelt wird, das 1 bis 35 Gew.-% Parfum beladene Polyamidteilchen, von denen mindestens 90 Gew.-% eine Teilchengröße im Bereich von 0,5 bis 50 Mikron, vorzugsweise etwa 1 bis etwa 25 Mikron, vor allem etwa 2 bis etwa 20 Mikron, aufweisen, enthält.
Die Bestimmung der Teilchengröße erfolgt auf dem Fachmann geläufige Weise, beispielsweise durch Sieb- oder Sedimentationsanlayse.
Die Art des verwendeten Parfumöls ist an sich zweitrangig; im Prinzip eignet sich jedes bekannte und in kosmetischen Mitteln eingesetzte Parfum für diesen Zweck.

JP5117134 beschreibt ein Verfahren zum dauerhaften Verformen von menschlichen Haaren, wobei auf das Haar in an sich bekannter Weise eine wässrige Reduktionsmittelzusammensetzung aufgebracht, anschliessend mit einer mindestens ein Oxidationsmittel enthaltenden Zusammensetzung fixiert wird (Siehe Zusammenfassung), dadurch gekennzeichnet, dass diese Zusammensetzung mit Parfum beladene Polyamidteilchen, von denen mindestens 90 Gew.-% eine Teilchengrösse im Bereich von 0,5 bis 50 Mikron aufweisen, enthält (Siehe Absätze [0011,0012,0015]).

Die Herstellung der parfumbeladenen Polyamidteilchen erfolgt durch Lösen des Parfumöls in einem geeigneten Lösungsmittel wie Wasser, Ethanol, n-Hexan etc.
Diese Lösung wird unter Rühren mit dem Polyamidpulver vermischt.
Anschließend wird das Lösungsmittel, je nach Flüchtigkeit der Parfumbestandteile unter geringer Erwärmung (etwa 25 bis maximal etwa 50°C) und/oder Vakuum entfernt.
Das Beladen von Polyamidpulver mit aktiven Materialien ist grundsätzlich bekannt und beispielsweise bei V. Parison, Cosmetics & Toiletries, Vol. 108 (Dezember 1993), S. 97-100, beschrieben.
Als Grundlage geeignete Polyamidpulver sind beispielsweise die unter den Handelsnamen "Orgasol^{R}" und "Nylon^{R}" bekannten Produkte, deren generische Bezeichnungen "Polyamide 6" oder "Polyamide 12" lauten.

Der bevorzugte Anteil derselben in den erfindungsgemäß eingesetzten Haarbehandlungsmitteln liegt zwischen etwa 0,1 bis etwa 10, vorzugsweise etwa 0,25 bis etwa 7,5, insbesondere etwa 0,5 bis etwa 5 Gew.-% der Zusammensetzung.

Die Menge an Parfum, mit denen die Polyamidpulverteilchen beladen sind, ist ebenfalls variabel und ist insbesondere von der Art des Parfums, der Teilchengröße des Polyamidpulvers sowie den Absorptionsbedingungen abhängig.

Sie kann zwischen etwa 1 bis etwa 35, vorzugsweise etwa 5 bis etwa 25, insbesondere etwa 10 bis etwa 20 Gew.-% Parfum an beladenen Teilchen liegen.

Die im ersten Schritt des erfindungsgemäßen Verfahrens verwendeten Verformungsmittel enthalten in der Regel eine reduzierende Thioverbindung.
Bevorzugt sind Thioglykolsäure und Thiomilchsäure sowie deren Salze, insbesondere die Ammonium- und Ethanolaminsalze.

Weitere einsetzbare Thioverbindungen sind insbesondere Cystein bzw. dessen Hydrochlorid, Homocystein, Cysteamin, N-Acetylcystein, Thioglycerin, Ethandiolmonothioglykolat, 1,2-Propylenglykolmonoglykolat (vgl. auch WO-A 93/1791), 1,3-Propandiolmonothioglykolat bzw. das daraus resultierende Isomerengemisch, 1,3-Butandiol- und 1,4-Butandiolmonothioglykolat bzw. deren Isomerengemische, Polyethylenglykol- wie Di-, Tri- und Tetraethlenglykolmonothioglykolate, Glycerinmonothiolactate und weitere Thiosäuren sowie deren Ester und Gemische derselben.

Auch die Verwendung anorganischer reduzierender Schwefelverbindungen wie Natriumhydrogensulfit ist prinzipiell möglich.

Der Gesamtgehalt an Reduktionsmitteln in der Verformungs-Zusammensetzungen beträgt üblicherweise 2,5 bis etwa 15 Gew.-%, berechnet auf freie Thioglykolsäure als Bezugssubstanz.

Die Reduktionsmittel enthaltenden Dauerwellpräparate können, falls erforderlich, einen Gehalt an Alkalisierungsmitteln aufweisen. Die Menge ist abhängig vom reduzierenden Wirkstoff und dem angestrebten pH-Wert der Zusammensetzung. Vorzugsweise enthält die Reduktionsmittel-Zusammensetzung etwa 0,1 bis etwa 5, insbesondere etwa 0,5 bis etwa 2,5 Gew.-% desselben.

Bevorzugte Alkalisierungsmittel sind Ammoniumcarbamat, Ammoniak und/oder Ammonium(bi)carbonat. Es wird die Einstellung eines pH-Wertes im Bereich zwischen etwa 6,5 und etwa 9,5, vorzugsweise etwa 7 bis 8,5, angestrebt.

Die im erfindungsgemäßen Verfahren zum Einsatz kommenden Dauerwellmittel sowie auch die Fixier- bzw. Nachbehandlungsmittel enthalten vorzugsweise auch Tenside. Deren Anteil liegt bei etwa 0,1 bis etwa 10, insbesondere etwa 1 bis etwa 5 Gew.-% der Gesamtzusammensetzung.

Sowohl bei den in den Reduktionsmittel-Zusammensetzungen als auch bei den in den Fixiermitteln eingesetzten Tensiden handelt es sich vorzugsweise um die bekannten anionaktiven Produkte, die gegebenenfalls auch in Kombination mit nichtionischen Tensiden zum Einsatz gelangen.

Geeignete anionische Tenside sind insbesondere die bekannten Alkylethersulfate und -carbonsäuren, insbesondere in Form ihrer Alkalisalze, sowie Eiweiß-Fettsäure-Kondensate.

Geeignete nichtionische Tenside sind vorzugsweise C₈-C₁₈-Fettalkoholpolyglykolether, Fettsäurepolyglykolester, Fettsäurealkanolamide, Aminoxide und vor allem C₈-C₁₈-Alkylpolyglucoside.

Es können auch amphotere Tenside wie die bekannten Betaine und Amidobetaine sowie, insbesondere in kationischen Fixierungen, kationaktive Tenside wie quaternäre Ammoniumverbindungen eingesetzt werden.

Ein weiterer wünschenswerter Bestandteil der im erfindungsgemäßen Verfahren verwendeten Reduktionsmittel-Zusammensetzungen ist ein C₃-C₈-Alkandiol bzw. dessen Ether, insbesondere Mono-C₁-C₃-alkylether.

Bevorzugte Substanzen sind in diesem Zusammenhang 1,2- und 1,3-Propandiol, 1-Methoxypropanol(-2), 1-Ethoxypropanol(-2), 1,3- und 1,4-Butandiol, Diethylenglykol und dessen Monomethyl- und Monoethylether sowie Dipropylenglykol und dessen Monomethyl- und Monoethylether.

Der Anteil dieser Diole liegt vorzugsweise zwischen 0,5 und 30, vorzugsweise etwa 1 bis etwa 15, insbesondere etwa 1 bis etwa 10 Gew.-% der Reduktionmittel-Zusammensetzung.

Neben den C₃-C₆-Alkandiolen bzw. deren Ethern können zusätzlich auch Monoalkohole wie Ehtanol, Propanol-1, Propanol-2 sowie Polyalkohole wie Glycerin und Hexantriol, Ethylcarbitol, Benzylalkohol, Benzyloxyethanol sowie Propylencarbonat (4-Methyl-1,3-dioxolan-2-on), N-Alkylpyrrolidone und Harnstoff Verwendung finden.

Weitere mögliche Bestandteile sind kationische, anionische, nichtionische und amphothere Polymere, vorzugsweise in einer Menge von etwa 0,1 bis etwa 5, insbesondere etwa 0,25 bis 2,5 Gew.-% der Gesamtzusammensetzung des Wellmittels.

Die erfindungsgemäß eingesetzten Mittel können selbstverständlich alle in Dauerwellmitteln üblichen Stoffe enthalten, auf deren detaillierte Aufzählung hier verzichtet wird, und als wäßrige Lösungen, Emulsionen, Cremes, Schäume etc. vorliegen.

Es kann sich dabei um einphasige Produkte oder um in getrennten Verpackungen untergebrachte Zusammensetzungen handeln, die bei der Anwendung vereinigt werden, wie sie z.B. in der DE-C 43 04 828 beschrieben sind.

Zur Vermeidung von Wiederholungen wird hierzu auf den Stand der Technik verwiesen, wie er beispielsweise in "Ullmanns's Encyclopedia of Industrial Chemistry", Vol A12 (1986), S. 599 bis 591, sowie insbesondere in der Monographie von K. Schrader, "Grundlagen und Rezepturen der Kosmetika", 2. Aufl. (1989, Hüthig-Verlag), S. 823 bis 840, sowie in dem Übersichtsartikel von D. Hollenberg et al. in "Seifen-Öle-Fette-Wachse", 117 (1991), S. 81-87, beschrieben ist.
Die dort geoffenbarten Zusammensetzungen und Einzelbestandteile, auf die ausdrücklich Bezug genommen wird, können auch im Rahmen der vorliegenden Erfindung verwendet werden.

Falls erwünscht, kann vor dem Auftrag des Reduktionsmittels noch ein Vorbehandlungsmittel appliziert werden, wie es beispielsweise in der DE-A 37 40 926 beschrieben ist. Nach dem Aufbringen dieses Vorbehandlungsmittels wird das Haar aufgewickelt und die Reduktionsmittel-Zusammensetzung aufgetragen. Nach etwa 15- bis 30-minütiger Einwirkung und Spülung erfolgt die Fixierung mit den bekannten Peroxid- oder Bromat-Zusammensetzungen.
Bevorzugt sind Wasserstoffperoxid-Zusammensetzungen, wobei der Gehalt an H₂O₂ etwa 0,05 bis 6 Gew.-%, vorzugsweise etwa 0,1 bis 5, insbesondere etwa 0,5 bis 4 Gew.-% beträgt.

Ebenso kann selbstverständlich auch eine an sich bekannte Zwischenbehandlung zwischen Reduktions- und Neutralisierungsphase erfolgen.

Bei den die mit Parfum beladenen Polyamidmikroteilchen enthaltenden Zusammensetzungen handelt es sich entweder um solche, die nach der Anwendung auf dem Haar verbleiben, sogenannte "Leave on"-Produkte wie Haarkuren, Haarfestiger, Styling-Produkte etc., oder um solche, die nach der Einwirkung wieder vom Haar entfernt werden, wie Haarwasch- und Haarspülmittel, Kurzzeit-Haarkuren etc., sogenannte "Rinse-off"-Präparate.

Haarkonditionierende Produkte wie Haarspülmittel, Kuren oder Styling-Präparate enthalten üblicherweise konditionierende Wirkstoffe.

Geeignete haarkonditionierende Wirkstoffe, deren Menge in den erfindungsgemäß verwendeten Mitteln üblicherweise bei etwa 0,25 bis etwa 15, insbesondere 0,5 bis etwa 10, vorzugsweise etwa 1 bis 7,5 Gew.-%, bezogen auf die Gesamtmenge des Mittels liegt, sind insbesondere kationische Tenside, vor allem quaternäre Ammoniumverbindungen.

Geeignete langkettige quaternäre Ammoniumverbindungen, die allein oder im Gemisch miteinander eingesetzt werden können, sind beispielsweise Cetyltrimethylammoniumchlorid, Dimethyldicetylammoniumchlorid, Trimethylcetylammoniumbromid, Dimethyldistearylammoniumchlorid, Stearyltrimethylammoniumchlorid, Dimethylstearylbenzylammoniumchlorid, Benzyltetradecylodimethylammoniumchlorid, Dimethyl- dihydriertes-Talgammoniumchlorid, Laurylpyridiniumchlorid, Lauryldimethylbenzylammoniumchlorid, Behenyltrimethylammoniumchlorid, Lauryltrimethylammoniumchlorid, Tris-(oligooxyethyl)alkylammoniumphosphat, Cetylpyridiniumchlorid, etc.

Gut geeignet sind auch die in der EP-A 472 107 geoffenbarten quaternären Ammoniumsalze.

Im Prinzip sind alle quaternären Ammoniumverbindungen, wie sie im jeweils gültigen "CTFA International Cosmetic Ingredient Dictionary" unter dem Trivialnamen "Quaternium" aufgeführt sind, einsetzbar.
Geeignet sind auch langkettige Amine, z. B. Stearamidopropyldimethylamin.

Der Anteil dieser obengenannten Verbindungen liegt vorzugsweise bei etwa 0,5 bis 10, insbesondere etwa 1 bis 7,5 Gew.-%, berechnet auf die Gesamtzusammensetzung.

Besonders geeignete langkettige quaternäre Ammoniumverbindungen sind Esterquats der allgemeinen Formel (I) 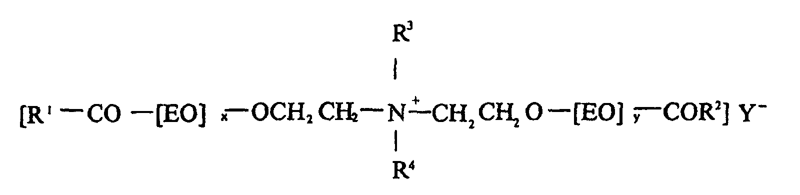 in der R¹ und R² für eine gegebenenfalls hydroxysubstituierte C₈-C₂₂-Alkyl- oder Alkenylgruppe, R³ und R⁴ für eine C₁-C₃-Alkylgruppe oder eine Gruppe -CH₂-CH₂-O-[EO]_{z}H sowie x,y und z für 0 bis 5 und Y⁻ für ein Anion stehen.

Ein besonders bevorzugtes Esterquat ist im Rahmen der Erfindung ein solches, in der die Reste R¹ und R² jeweils eine Oleylgruppe oder eine C₁₂-C₁₈-Alkylgruppe, der Rest R³ eine Methylgruppe und der Rest R⁴ eine Gruppe -CH₂-CH₂-O-[EO]_{z}-H bedeuten.

Das Anion Y⁻ ist vorzugsweise ein Halogenid wie Cl⁻ oder Br⁻, ein niederes Alkylsulfat, z.B. Methosulfat und Ethosulfat, oder ein Alkylphosphat, jedoch können selbstverständlich auch andere Reste eingesetzt werden.

Diese Verbindungen sind an sich bekannt und beispielsweise unter den Handelsnamen "Schercoquat^{A}", "Dehyquart^{R}F30" und "Tetranyl^{R}" im Handel.

Der Einsatz dieser Verbindungen in Haarpflegemitteln ist ebenfalls bereits bekannt und beispielsweise in der WO-A 93/10748, der WO-A 92/06899 und der WO-A 94/16677 beschrieben.

Ebenso geeignet sind "Amidoquats" der allgemeinen Formel 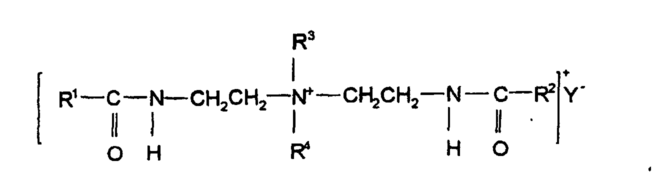 in der R¹ und R² jeweils für eine gegebenenfalls hydroxysubstituierte C₈-C₂₂-Alkyl- oder Alkenylgruppe, R³ und R⁴ für eine C₁-C₃-Alkylgruppe oder eine Gruppe -CH₂-CH₂-O-[EO]ₓ-H, sowie x für 0 bis 5, Y⁻ für ein Anion stehen.

Bevorzugte Reste R¹ und R² sind C₁₂-C₁₈-Alkyl- und Oleylreste, der Rest R³ eine Methylgruppe und der Rest R⁴ eine Gruppe -CH₂-CH₂-O[EO]ₓ-H, worin x 0 bis 3 ist; Y⁻ ist vorzugsweise ein Methosulfat-, Ethylsulfat-, Chlorid oder Phosphatanion.

Diese Verbindungen sind an sich bekannt und beispielsweise unter den Markenbezeichnungen "INCROQUAT® HO" oder "OCS" im Handel.

Weitere geeignete haarkonditionierende Wirkstoffe sind synthetische oder natürliche haarkonditionierende Polymere, vorzugsweise in einer Menge von 0,1 bis 10, insbesondere 0,25 bis 5 Gew.-% der Gesamtzusammensetzung.

Besonders bevorzugt sind hierbei die unter der CTFA-Bezeichnung "Polyquaternium" bekannten kationischen (Co-)Polymeren, jedoch können auch nichtionische, anionische und/oder amphotere Polymere, beispielsweise solche vom Typ "Amphomer^{R}", alleine oder im Gemisch verwendet werden.

Weitere haarkonditionierende Wirkstoffe sind lipophile, d.h. fett- und ölhaltige Substanzen, zu denen auch Wachse zählen, sind insbesondere natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, oder auch Oliven-bzw. Sojaöl, Lanolin und dessen Derivate, ebenso Mineralöle wie Paraffinöl und Vaseline.
Synthetische Öle und Wachse sind beispielsweise Silikonöle, Polyethylengykole, etc.
Weitere geeignete hydrophobe Komponenten sind insbesondere Fettalkohole, vorzugsweise solche mit etwa 8 bis 22 Kohlenstoffatomen im Molekül wie Myristyl-, Cetyl-, Stearylalkohol, Wachsalkohole und Fettsäureester wie Isopropylmyristat, -palmitat, -stearat und -isostearat, Oleyloleat, Isocetylstearat, Hexyllaurat, Dibutyladipat, Dioctyladipat, Myristylmyristat, Oleylerucat, Polyethylenglykol- und Polyglycerylfettsäureester wie PEG-7-glycerylcocoat, Cetylpalmitat, etc.

Diese hydrophoben Komponenten sind in den erfindungsgemäß verwendeten Zusammensetzungen vorzugsweise in einer Gesamtmenge von etwa 0,5 bis etwa 15, insbesondere etwa 1 bis 10, vor allem etwa 1,5 bis 7,5 Gew.-%, berechnet auf die Gesamtzusammensetzung, enthalten.

Weitere geeignete haarkonditionierende Zusatzstoffe sind Ceramide der allgemeinen Formel 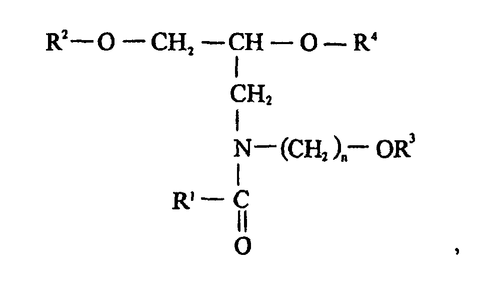 worin R¹ und R² gleiche oder verschiedene Alkyl- bzw. Alkenylreste mit 10 bis 22 Kohlenstoffatomen bedeuten, R³ für Wasserstoff oder eine Methyl-, Ethyl-, n-Propyl- oder Isopropylgruppe steht, R⁴ Wasserstoff, eine Hydroxymethyl-,Hydroxyethyl-, Dihydroxyethyl- oder Dihydroxypropylgruppe, und n eine ganze Zahl von 1 bis 6 bedeuten, insbesondere der Art, wie sie aus der EP 227 994 A1 und der WO-A 96/37462 bekannt sind, jedoch sind auch andere Ceramide, beispielsweise die aus der WO-A 97/15724 oder der EP 647 617 B1 bekannten Ceramide, geeignet.

Die bevorzugten Gruppen R¹ und R² sind C₁₂-C₁₈-Alkylreste; n ist eine Zahl von 1 bis 3, R³ bedeutet vorzugsweise Wasserstoff oder einen Methylrest, und R⁴ Wasserstoff oder einen Dihydroxypropylrest.
Besonders bevorzugt sind Verbindungen, in denen R¹ einen C₁₂-C₂₄-Alkylrest, insbesondere eine C₁₃H₂₇-Alkylgruppe, R² einen C₁₄-C₁₈-Alkylrest, insbesondere eine C₁₆H₃₃-Alkylgruppe, R³ einen Methylrest, R⁴ eine 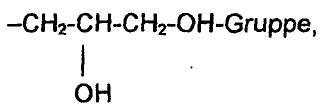 und n 3 darstellen, oder eine Verbindung, wo R¹ für einen C₁₅-C₃₁-Alkylrest, R² für einen C₁₆H₃₃-Alkylrest, R³ und R⁴ für je ein Wasserstoffatom und n für 2 stehen.

Deren Menge in den erfindungsgemäß eingesetzten Haarnachspülmitteln liegt zweckmäßigerweise bei etwa 0,01 bis 10, vorzugsweise etwa 0,05 bis 7,5, insbesondere etwa 0,1 bis 5 Gew.-%, berechnet auf die Gesamtzusammensetzung.

Weitere geeignete haarkonditionierende Wirkstoffe sind wasserunlösliche Vitamine und deren Derivate, beispielsweise Vitamin E und dessen Ester wie Tocopherolacetat, -propionat, -palmitat, etc.

Weitere Zusatzstoffe, deren Art und Menge natürlich von der Applikationsform des Mittels abhängig sind, sind Fette, Fettalkohole, Emulgatoren, pH-Regulatoren, Lösungs- und Verdünnungsmittel, Lösungsvermittler, Konservierungsmittel, Parfums, etc.

Als Tenside sind insbesondere nichtionische Tenside geeignet.
Besonders bevorzugte nichtionische Tenside sind die bekannten C₈-C₁₈-Alkylpolyglucoside, insbesondere mit einem Polykondensationsgrad von 1,2 bis 3, vor allem solche der allgemeinen Formel

R-O-(R¹O)ₙ-Zₓ,

worin R eine Alkylgruppe mit 8 bis 18 Kohlenstoffatomen, R¹ eine Ethylen- oder Propylengruppe, Z. einen Saccharidrest mit 5 bis 6 Kohlenstoffatomen, n eine Zahl von 0 bis 10 und x eine Zahl zwischen 1 und 2,5 bedeuten, in einer Menge von 1 bis 10, insbesondere 2,5 bis 7,5 Gew.-% der Gesamtzusammensetzung.

Andere geeignete nichtionische Tenside sind beispielsweise die verschiedenen Sorbitanester, wie Polyethylenglykolsorbitanstearinsäureester, Fettsäurepolyglykolester oder auch Mischkondensate aus Ethylenoxid und Propylenoxid, wie sie beispielsweise unter der Handelsbezeichnung "Pluronics" im Verkehr sind.
Daneben sind natürlich auch anionische und/oder amphotere bzw. zwitterionische Tenside geeignet.

Die erfindungsgemäß verwendeten haarkonditionierenden Mittel liegen vorzugsweise als wäßrige oder wäßrig/alkoholische Lösung, wäßrige Emulsion, Mikroemulsion, Dispersion oder opakes oder transparentes Gel vor und können auch als Aerosole konfektioniert werden. Solche Zusammensetzungen und ihre Herstellung sind dem Fachmann grundsätzlich bekannt und bedürfen daher keiner näheren Erläuterung.

Der pH-Wert der erfindungsgemäß verwendeten Haarnachbehandlungsmittel ist nicht kritisch; er kann vorzugsweise bei 3 bis etwa 8, insbesondere zwischen 4 und 6,5, liegen.

Ebenso liegt die Viskosität im allgemein üblichen Bereich und ist selbstverständlich abhängig von der Darreichungsform des Mittels.

Die auf dem Haar verbleibenden "Leave on"-Produkte sind im Prinzip gleich zusammengesetzt.

Falls es sich dabei um Styling-Produkte oder haarfestigende Präparate handelt, enthalten diese selbstverständlich auch mindestens einen polymeren Filmbildner.

Diese sind dem Fachmann bekannt und werden im folgenden im Detail diskutiert.

Falls es sich bei dem Nachbehandlungsmittel um ein Haarwaschmittel handelt, enthält dieses die in Shampoos üblichen Stoffe.

Solche sind insbesondere Tenside, bevorzugt anionische Tenside, in einer Menge von etwa 5 bis etwa 50 Gew.-%, vorzugsweise etwa 10 bis etwa 25 Gew.-% der Zusammensetzung.

Geeignete anionaktive Tenside im Rahmen der Erfindung sind solche vom Sulfat-, Sulfonat-, Carboxylat- und Alkylphosphat-Typ, vor allem natürlich diejenigen, die in Shampoos üblicherweise zum Einsatz gelangen, beispielsweise die bekannten C₁₀-C₁₈-Alkylsulfate und insbesondere die entsprechenden Ethersulfate, beispielsweise C₁₂-C₁₄-Alkylethersulfat, Laurylethersulfat, insbesondere mit 1 bis 4 Ethylenoxidgruppen im Molekül, weiterhin Monoglycerid(ether)sulfate, Fettsäureamidsulfate, die durch Ethoxylierung und anschließende Sulfatierung von Fettsäurealkanolamiden erhalten werden, und deren Alkalisalze sowie Salze langkettige Mono- und Dialkylphosphate, die milde, hautverträgliche Detergentien darstellen.

Weiterhin geeignete anionische Tenside sind α-Olefinsulfonate bzw. deren Salze und insbesondere Alkalisalze von Sulfobernsteinsäurehalbestem, beispielsweise das Dinatriumsalz des Monooctylsulfosuccinats und Alkalisalze langkettiger Monoalkylethoxysulfosuccinate.

Geeignete Tenside vom Carboxylat-Typ sind Alkylpolyethercarbonsäuren und deren Salze der Formel

R - (C₂H₄O)ₙ - O - CH₂ COOX,

worin R eine C₈-C₂₀-Alkylgruppe, vorzugsweise eine C₁₂-C₁₄-Alkylgruppe, n eine Zahl von 1 bis 20, vorzugsweise 2 bis 17, und X H oder vorzugsweise ein Kation der Gruppe Natrium, Kalium, Magnesium und Ammonium, das gegebenenfalls hydroxyalkylsubstituiert sein kann, bedeuten, sowie Alkylamidopolyethercarbon-säuren der allgemeinen Formel 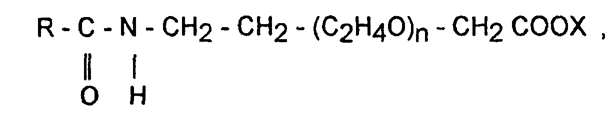 worin R und X die vorstehend angegebene Bedeutung haben, und n insbesondere für eine Zahl von 1 bis 10, vorzugsweise 2,5 bis 5, steht.

Derartige Produkte sind seit längerem bekannt und im Handel, beispielsweise unter den Handelsnamen "AKYPO®" und "AKYPO-SOFT®".

Auch C₈-C₂₀-Acylisethionate können, allein oder im Gemisch mit anderen Tensiden, eingesetzt werden, ebenso Sulfofettsäuren und deren Ester.
Es können auch Mischungen aus mehreren anionischen Tensiden eingesetzt werden, beispielsweise ein Gemisch aus einem α-Olefinsulfonat und einem Sulfosuccinat, vorzugsweise im Verhältnis von 1 : 3 bis 3 : 1, oder einem Ethersulfat und einer Polyethercarbonsäure oder Alkylamidoethercarbonsäure.

Eine Übersicht über die in flüssigen Körperreinigungsmitteln zum Einsatz gelangenden anionaktiven Tenside findet sich im übrigen in der Monographie von K.Schrader, "Grundlagen und Rezepturen der Kosmetika", 2. Aufl. (1989, Hüthig Buchverlag), S. 595-600 und S. 683 bis 691.
Weitere geeignete anionische Tenside sind auch C₈-C₂₂-Acylaminocarbonsäuren bzw. deren wasserlösliche Salze, vorzugsweise in einer Menge von 0,5 bis 10, insbesondere 1 bis 7,5 Gew.-%, berechnet auf die Gesamtzusammensetzung. Besonders bevorzugt sind N-Lauroylglutamat, insbesondere als Natriumsalz, sowie beispielsweise N-Lauroylsarcosinat, N-C₁₂-C₁₈-Acylasparaginsäure, N-Myristoylsarcosinat, N-Oleoylsarcosinat, N-Lauroylmethylalanin, N-Lauroyllysin und N-Lauroylaminopropylglycin, vorzugsweise in Form ihrer wasserlöslichen Alkali- oder Ammonium-, insbesondere Natriumsalze, vorzugsweise im Gemisch mit den obengenannten anionaktiven Tensiden.

Der besonders bevorzugte Mengenbereich an anionischen Tensiden in den erfindungsgemäß verwendeten flüssigen Shampoos liegt zwischen etwa 5 und etwa 35 Gew.-%, insbesondere bei etwa 7,5 bis etwa 25 Gew.-%, besonders bevorzugt bei etwa 10 bis etwa 20 Gew.-%, berechnet auf die Gesamtzusammensetzung des Mittels.

Weitere geeignete Tenside in den erfindungsgemäß verwendeten Haarwaschmitteln sind nichtionische Tenside, vorzugsweise im Gemisch mit anionaktiven und/oder zwitterionischen bzw. amphoteren Tensiden.
Diese sind bei Schrader, I.c., auf den Seiten 600-601 und S. 694-695 beschrieben. Geeignet sind insbesondere Alkylpolyglucoside mit der allgemeinen Formel

R-O-(R¹O)ₙ-Zₓ,

worin R eine Alkylgruppe mit 8 bis 18 Kohlenstoffatomen, R¹ eine Ethylen- oder Propylengruppe, Z einen Saccharidrest mit 5 bis 6 Kohlenstoffatomen, n eine Zahl von 0 bis 10 und x eine Zahl zwischen 1 und 5 bedeuten.
Diese Alkylpolyglucoside sind in letzter Zeit insbesondere als ausgezeichnete hautverträgliche schaumverbessernde Mittel in flüssigen Wasch- und Körperreinigungsmitteln bekannt geworden und sind in einer Menge von 1 bis 20, insbesondere 2,5 bis 10 Gew.-%, der Gesamtzusammensetzung enthalten. Gemische aus anionaktiven Tensiden und Alkylpolyglucosiden sowie deren Verwendung in flüssigen Körperreinigungsmitteln sind an sich bereits bekannt, beispielsweise aus der EP-A 70 074. Die dort beschriebenen Alkypolyglucoside sind prinzipiell auch im Rahmen der vorliegenden Erfindung geeignet; ebenso die aus der EP-A 358 216 bekannten Gemische aus Sulfosuccinaten und Alkylpolyglucosiden.

Weitere nichtionische Tensidbestandteile sind beispielsweise langkettige Fettsäuremono- und -dialkanolamide, wie Cocosfettsäuremonoethanolamid und Myristinfettsäuremonoethanolamid, die auch als Schaumverstärker eingesetzt werden können.

Andere zusätzlich mitverwendbare nichtionische Tenside sind z.B. die verschiedenen Sorbitanester, wie Polyethylenglykolsorbitanstearinsäureester, Fettsäurepolyglykolester oder auch Mischkondensate aus Ethylenoxid und Propylenoxid, wie sie beispielsweise unter der Handelsbezeichnung "Pluronics" im Verkehr sind. Weitere zusätzlich einsetzbare Tenside sind Aminoxide in einer Menge von etwa 0,25 bis etwa 5, vorzugsweise etwa 0,5 bis etwa 3,5 Gew.-%, berechnet auf die Gesamtzusammensetzung des Mittels.
Solche Aminoxide gehören seit langem zum Stand der Technik, beispielsweise C₁₂-C₁₈-Alkyldimethylaminoxide wie Lauryldimethylaminoxid, C₁₂-C₁₈-Alkylamidopropyl- oder -ethylaminoxide, C₁₂-C₁₈-Alkyldi(hydroxyethyl)- oder -(hydroxypropyl)aminoxide, oder auch Aminoxide mit Ethylenoxid- und /oder Propylenoxidgruppen in der Alkylkette.
Aminoxide sind beispielsweise unter den Bezeichnungen "Ammonyx® ", "Aromox® " oder "Genaminox®" im Handel.
Die erfindungsgemäß verwendeten Zusammensetzungen können als weiteren Tensid-Bestandteil amphotere bzw. zwitterionische Tenside, beispielsweise in einer Menge von etwa 0,5 bis etwa 10, vorzugsweise von etwa 1 bis etwa 7,5 Gew.-%, bezogen auf die Gesamtzusammensetzung, enthalten, ebenfalls vorzugsweise im Gemisch mit anionischen und/oder nichtionischen Tensiden.
Als solche sind insbesondere die verschiedenen bekannten Betaine wie Fettsäureamidoalkylbetaine und Sulfobetaine, beispielsweise Laurylhydroxysulfobetain, zu nennen; auch langkettige Alkylaminosäuren wie Cocoaminoacetat, Cocoaminopropionat und Natriumcocoamphopropionat und -acetat haben sich als geeignet erwiesen.

Im einzelnen können Betaine der Struktur 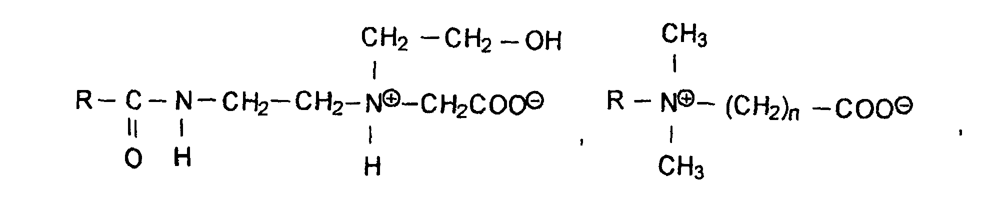 wobei R eine C₈-C₁₈-Alkylgruppe und n 1 bis 3 bedeuten,
Sulfobetaine der Struktur 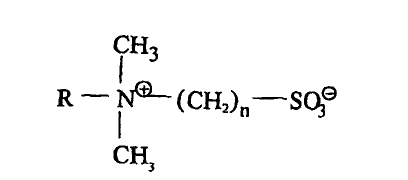 wobei R eine C₈-C₁₈-Alkylgruppe und n 1 bis 3 bedeuten,
und Amidoalkylbetaine der Struktur 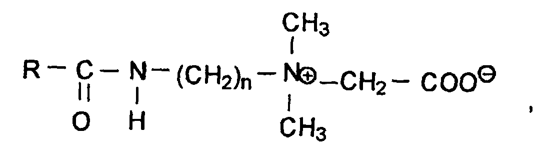 wobei R eine C₈-C₁₈-Alkylgruppe und n 1 bis 3 bedeuten, verwendet werden.

Die erfindungsgemäß verwendeten Shampoos können vorzugsweise auch konditionierende Wirkstoffe wie Eiweißhydrolysate und Polypeptide, z.B., Keratinhydrolysate, Kollagenhydrolysate vom Typ "Nutrilan^{R}" oder Elastinhydrolysate sowie insbesondere auch pflanzliche, gegebenenfalls kationisierte Eiweißhydrolysate, z.B. "Gluadin^{R}", enthalten.

Weitere bevorzugte mögliche Zusätze sind haarkonditionierende Polymere. Diese können nichtionische Polymere, vorzugsweise alkohol- und/oder wasserlösliche Vinylpyrrolidon-Polymere wie ein Vinylpyrrolidon-Homopolymerisat oder -Copolymerisat, insbesondere mit Vinylacetat, sein.
Geeignete Vinylpyrrolidon-Polymere sind z.B. die unter dem Handelsnamen "Luviskol®" bekannten Produkte, beispielsweise die Homopolymerisate "Luviskol® K 30, K 60 und K 90" sowie die wasser- bzw. alkohollöslichen Copolymerisate aus Vinylpyrrolidon und Vinylacetat, die unter dem Handelsnamen "Luviskol® VA 55 bzw. VA 64" von der BASF AG vertrieben werden.
Weitere geeignete nichtionische Polymere sind Vinylpyrrolidon/Vinylacetat/Vinylpropionat-Copolymere wie "Luviskol® VAP 343", Vinylpyrrolidon/(Meth)Acrylsäureester-Copolymere sowie Chitosan-Derivate.
Ihr Anteil in den erfindungsgemäßen Haarwaschmitteln liegt, wenn vorhanden, zwischen etwa 0,05 und etwa 5, vorzugsweise 0,1 und 2,5, insbesondere etwa 0,15 bis 1,5 Gew.-%, bezogen auf die Gesamtzusammensetzung des Mittels.

Anstelle oder zusätzlich zu den nichtionischen Polymeren können als haarkonditionierende Polymere auch kationische und/oder anionische und/oder amphotere Polymere in den genannten Mengen eingesetzt werden.

Bevorzugte haarkonditionierende kationische Polymere sind die altbekannten quaternären Cellulosederivate des Typs "Polymer JR" sowie quaternisierte Homo- und Copolymere des Dimethyldiallylammoniumchlorids, wie sie unter dem Handelsnamen "Merquat®" im Handel sind, quaternäre Vinylpyrrolidon-Copolymere, insbesondere mit Dialkylaminoalkyl(meth)acrylaten, wie sie unter dem Namen "Gafquat®" bekannt sind, Copolymerisate aus Vinylpyrrolidon und Vinylimidazoliniummethochlorid, die unter dem Handelsnamen "Luviquat®" angeboten werden, Polyamino-Polyamid-Derivate, beispielsweise Copolymere von Adipinsäure-Dimethylaminohydroxypropyldiethylentriamin, wie sie unter dem Namen "Cartaretine® F" vertrieben werden, sowie auch bisquaternäre langkettige Ammoniumverbindungen der in der US-PS 4 157 388 beschriebenen Harnstoff-Struktur, die unter dem Handelsnamen "Mirapol® A 15" im Handel sind.

Verwiesen wird in diesem Zusammenhang auch auf die in den DE-OSen 25 21 960, 28 11 010, 30 44 738 und 32 17 059 genannten kationaktiven Polymeren sowie die in der EP-A 337 354 auf den Seiten 3 bis 7 beschriebenen Produkte. Es können auch Mischungen verschiedener kationischer Polymerer eingesetzt werden.

Zu den kationischen Polymeren zählen auch die in der EP-A 524 612 und der EP-A 640 643 beschriebenen Quaternisierungsprodukte aus Pfropfpolymerisaten von Organopolysiloxanen und Polyethyloxazolinen.

Als amphotere Polymere, die allein oder im Gemisch mit mindestens einem weiteren kationischen, nichtionischen oder anionischen Polymeren zum Einsatz gelangen, seien insbesondere Copolymerisate aus N-Octylacrylamid, (Meth)Acrylsäure und tert.-Butylaminoethylmethacrylat vom Typ "Amphomer®"; Copolymerisate aus Methacryloylethylbetain und Alkylmethacrylaten vom Typ "Yukaformer®", z. B. das Butylmethacrylat-Copolymere "Yukaformer® Am75"; Copolymerisate aus Carboxylgruppen und Sulfongruppen enthaltenden Monomeren, z. B. (Meth)Acrylsäure und Itaconsäure, mit basischen Gruppen, insbesondere Aminogruppen, enthaltenden Monomeren wie Mono- bzw. Dialkylaminoalkyl(meth)acrylaten bzw. Mono- bzw. Dialkylaminoalkyl(meth)acrylamiden; Copolymere aus N-Octylacrylamid, Methylmethacrylat, Hydroxypropylmethacrylat, N-tert.-Butylaminoethylmethacrylat und Acrylsäure sowie die aus der US-A 3,927,199 bekannten Copolymeren genannt.

Geeignete anionische Polymere sind Vinylalkylether-, insbesondere Methylvinylether/Maleinsäure-Copolymere, die durch Hydrolyse von Vinylether/Maleinsäureanhydrid-Copolymeren entstehen und unter der Handelsbezeichnung "Gantrez® AN oder ES" vertrieben werden. Diese Polymeren können auch teilverestert sein, beispielsweise "Gantrez® ES 225", der Ethylester eines Ethylvinylethers/Maleinsäure-Copolymers, oder der Butyl- oder Isobutylester desselben.
Weitere geeignete anionische Polymere sind insbesondere Vinylacetat/Crotonsäureoder Vinylacetat/Vinylneodecanoat/Crotonsäure-Copolymere des Typs "Resyn®"; Natriumacrylat/Vinylalkohol-Copolymere des Typs "Hydagen® F", Natriumpolystyrolsulfonat, z.B. "Flexan® 130"; Ethylacrylat/Acrylsäure/N-tert.-Butylacrylamid-Copolymere des Typs "Ultrahold®"; Vinylpyrrolidon/Vinylacetat/Itaconsäure-Copolymere, Acrylsäure/Acrylamid-Copolymere bzw. Natriumsalze derselben vom Typ "Reten®"; etc.
Diese Polymeren eignen sich allein oder im Gemisch, auch zur Verwendung in haarfestigenden Mitteln.

Die erfindungsgemäß eingesetzten Shampoos können selbstverständlich alle üblichen, in solchen Mitteln zum Einsatz gelangenden Stoffe enthalten.

Als solche seien beispielhaft Komplexbildner, Farbstoffe, Konservierungsmittel, pH-Regler, Viskositätsregler wie anorganische Salze, soweit sie nicht ohnehin in den Tensid-Ausgangsmischungen enthalten sind, Duftstoffe, Perlglanzmittel, Verdickungsmittel, Feuchthaltemittel, pflanzliche und tierische Öle, wie sie weiter oben aufgezählt sind, genannt.
Eine Auflistung solcher Zusatzstoffe findet sich ebenfalls bei Schrader, l.c., auf S.695 bis 722.

Ein weiterer bevorzugter Bestandteil ist Ethoxydiglykol, vorzugsweise in einer Menge von 0,1 bis 5 Gew.-% des erfindungsgemäß verwendeten Shampoos.

Schließlich können auch noch bekannte Polysiloxane als konditionierende Mittel in den erfindungsgemäß verwendeten Nachbehandlungsmitteln mitverwendet werden. Deren bevorzugter Anteil liegt dabei etwa zwischen 0,5 und etwa 5, insbesondere 1 bis 3 Gew.-% der Gesamtzusammensetzung. Geeignet sind sowohl leichtflüchtige als auch schwerflüchtige cyclische oder lineare Polysiloxane, beispielsweise die unter dem Trivialnamen "Dimethicone" bzw. "Phenyldimethicone" sowie "Cyclomethicone" bekannten Silikonöle.
Geeignet sind beispielsweise auch die in der EP-A 398 177 beschriebenen Silikonderivate, die dort in Kombination mit Alkylpolyglucosiden in flüssigen Detergens-Zusammensetzungen eingesetzt werden.

Gemäß einer weiteren Ausführungsform der Erfindung können die erfindungsgemäß verwendeten Nachbehandlungsmittel wie Shampoos auch Farbstoffe zur direkten oder oxidativen Färbung von Haaren enthalten, z.B. Tönungs- oder Färbeshampoos. Besonders geeignet sind direktziehende Farbstoffe enthaltende Nachbehandlungsmittel.
Dadurch kann die Dauerwellung mit einer Färbung kombiniert werden.

Der pH-Wert der erfindungsgemäß eingesetzten Shampoos liegt im üblichen Bereich zwischen etwa 5 und 8,5; für Spezialprodukte kann er auch unterhalb 5 eingestellt werden.
Die Viskosität liegt ebenfalls im üblichen Bereich zwischen etwa 500 und etwa 50000 mPa.s bei 20°C, vorzugsweise etwa 1000 bis etwa 25000, insbesondere 2500 bis 10000 -mPa.s bei 20°C, gemessen nach Brookfield oder Höppler bei einer Scherspannung von 10 sec⁻¹.

Diese Zusammensetzungen können auch als Aerosol-Shampoos mit den üblichen Treibmitteln in Aluminiummonoblockdosen als auch Weißblechdosen, vorzugsweise mit Innenbeschichtung, z.B. auf Epoxyharz-Basis, versehen, als auch Kunststoffdosen abgepackt werden.
Die Herstellung der Produkte erfolgt durch Zusammenrühren der einzelnen Komponenten in Wasser, wobei auch Vormischungen verschiedener Bestandteile verwendet werden können.

Eine weitere Möglichkeit der Realisierung der Erfindung besteht in der Anwendung einer die mit Parfum beladenen Polyamidteilchen enthaltenden Zusammensetzung als Zwischenbehandlungsmittel, das heißt, die Aufbringung eines solchen Produkts nach dem Ausspülen des überschüssigen Reduktionsmittels, aber vor dem Aufbringen des Fixier- bzw. Neutralisationsmittels, wodurch ebenfalls eine Geruchsverbesserung erreicht wird.

Die folgenden Beispiele dienen der Illustration der Erfindung.

### Beispiel 1

### Dauerwelle für normales Haar

| | |
|---|---|
| Ammoniumthioglykolat (50%-ig) | 21,60 (Gew.-%) |
| Ammoniumhydrogencarbonat | 5,00 |
| Kationisches Polymer (Polyquaternium-11) | 1,00 |
| Nichtionischer Emulgator (Hydrierter Ricinusölpolyolester) | 0,80 |
| Ethoxydiglykol | 1,50 |
| Chlorophyllin | 0,05 |
| Dimethicone Copolyol Meadowfoamate | 0,40 |
| Entschäumer, Trübungsmittel, Parfum | q.s. |
| Ammoniak pH | 8,5 |
| Wasser ad | 100,0 |

Die Dauerwellung erfolgte durch 20- bis 30-minütige Einwirkung auf das auf Lockenwickler gewickelte Haar, Ausspülen und anschließende fünfminütiger Fixierung mit der folgenden Zusammensetzung:

| | |
|---|---|
| Wasserstoffperoxid | 2,5 (Gew.-%) |
| Cetylstearylalkohol | 2,0 |
| Natriumlauryethersulfat | 1,2 |
| C₁₂-C₁₄-Alkylpolyglykolether | 1,0 |
| Stabilisator | q.s. |
| Parfumöl | 0,5 |
| Wasser ad | 100,0 |

Nach Entfernen der Wickler, weiterer fünfminütiger Einwirkung, Waschen mit einem Shampoo und Trocknen wurde eine ausdrucksvolle Dauerwelle erzielt, die eine gepflegte Struktur und eine ausgezeichnete Naß- und Trockenkämmbarkeit zeigte.

Das Haar wies jedoch einen unangenehmen Geruch nach Thioverbindungen auf.

Die Zusammensetzung des verwendeten Shampoos war die folgende:

| | |
|---|---|
| Natriumalkylethersulfat (∼2,5 EO) | 10,0 (Gew.-%) |
| Natriumlauroylglutamat | 2,5 |
| Cocoamidopropylbetain | 2,0 |
| PEG-3-distearat | 2,0 |
| Polyquaternium-7 | 1,0 |
| Natriumchlorid | 1,2 |
| Parfum | 0,4 |
| Citronensäure | 0,1 |
| Panthenol | 0,3 |
| Natriumbenzoat | 0,5 |
| Wasser ad | 100,0 |

Waschen mit dem gleichen Shampoo, wobei aber das Parfum durch 2,0 Gew.-% eines mit 20 Gew.-% des gleichen Parfum beladenen Polyamidpulvers (Nylon^{R} 12; mittl. Teilchendurchmesser > 90% 15 bis 20 µm) ersetzt war, führte zu einem wesentlich besser riechenden Haar, der längere Zeit anhielt.

### Beispiel 2

### Zweiphasen-Produkt (Neutral-Dauerwelle)

| **Teil A:** | |
|---|---|
| Ammoniumhydrogencarbonat | 4,5 (g) |
| 1,2-Propandiol | 1,0 |
| Dimethicone Copolyol Meadowfoamate | 0,4 |
| Kationisches Polymer (Polyquatemium-22) | 1,0 |
| Cocoamidopropylbetain | 1,0 |
| Nichtionischer Lösungsvermittler | 0,8 |
| Parfum, Trübungsmittel | q.s. |
| Wasser ad | 72,0 |
| eingestellt mit NH₃ auf pH 8,6 | |

| **Teil B:** | |
|---|---|
| Ammoniumthioglykolat, 70%-ig | 18,0 (g) |
| Thiomilchsäure | 2,0 |
| 1,2-Propandiol | 0,5 |
| Wasser ad | 28,0 |
| eingestellt mit NH₃ auf pH 5,5 | |

Die Teilzusammensetzungen A und B wurden getrennt in ein bekanntes Zweikammerbehältnis eingebracht und unmittelbar vor der Anwendung auf das Haar durch Zerstören der Trennwand vereinigt, wobei ein Produkt mit einem pH-Wert von 7,4 erhalten wurde.

Die Dauerwellung erfolgte entsprechend dem in Beispiel 1 beschriebenen Verfahren, wobei die Haarwäsche nach der Fixierung mit einem Shampoo der folgenden Zusammensetzung erfolgte:

| | |
|---|---|
| Natriumlaurylethersulfat (∼2 EO) | 8,5 (Gew.-%) |
| Cocoamphoacetat | 3,0 |
| C₁₂-C₁₄-Alkylpolyglucosid (P.D.: ∼1,5) | 2,0 |
| Acrylates/Aminoacrylates/C₁₀-C₃₀-Alkyl-PEG 20-ltaconate | 2,5 |
| Copolymer | |
| PEG-60 hydriertes Ricinusöl | 0,5 |
| Mit 10 Gew.-% Parfum beladenes Polyamidpulver (Nylon^{R} | 5,0 |
| 12; mittl. Teilchengröße > 90% 15 bis 20 µm) | |
| Citronensäure | 1,0 |
| Natriumbenzoat | 0,5 |
| Wasser ad | 100,0 |

Das verwendete Fixiermittel besaß folgende Zusammensetzung:

| | |
|---|---|
| Wasserstoffperoxid | 4,0 (Gew.-%) |
| Quaternium-36 | 2,0 |
| Citronensäure | 0,2 |
| Acetanilid | 0,2 |
| Parfumöl | 0,5 |
| Wasser ad | 100,0 |

Es wurde ein nach dem Trocknen weitgehend von unangenehmem Geruch freies, dauergewelltes Haar erhalten.

Ersatz des parfumbeladenen Polyamidpulvers im Shampoo durch 0,5% des gleichen freien Parfums ergab einen unangenehmen Geruch nach Schwefelverbindungen.

### Beispiel 3

### Dauerwell-Zwischenbehandlungsmittel

| | |
|---|---|
| Lauryldimethicone Copolyol | 0,5 (Gew.-%) |
| Magnesiumsulfat • 7H₂O | 8,0 |
| Weizenproteinhydrolysat | 0,5 |
| Citronensäure • H₂O | 0,5 |
| Glutaminsäure | 0,5 |
| Nichtionischer Lösungsvermittler | 0,6 |
| Parfum | 0,3 |
| Ammoniak pH | 4,5 |
| Wasser ad | 100,0 |

Diese Zusammensetzung wurde zwischen dem Reduktions- und Fixiervorgang mittels handelsüblicher Dauerwell- und Fixiermittel aufgebracht, 5 Minuten einwirken gelassen und anschließend ausgespült.

Das dauergewellte Haar wies den üblichen Thiogeruch auf.

Ersatz des Parfums durch 3,0 Gew.-% eines mit 10 Gew.-% des gleichen Parfums beladenen Polyamidpulvers (ORGASOL^{R} 2002; mittl. Teilchengröße 5 µm) führte zu einem deutlich verbessertem Geruch des dauergewellten Haares.

### Beispiel 4

Auf entsprechend dem Verfahren nach Beispiel 1 dauergewelltes Haar wurde nach dem Fixieren ein **Styling-Gel** der folgenden Zusammensetzung:

| | |
|---|---|
| Tocopherylacetat | 0,5 (Gew.-%) |
| Mit 20 Gew.-% beladenes Polyamidpulver | 1,5 |
| (mittl. Teilchengröße: > 90 Gew.-% zwischen | |
| 4 und 12 µm) | |
| Carbopol^{R} ETD 2001 (Carbomer) | 0,5 |
| Ethanol | 11,0 |
| Vinylpyrrolidon/Vinylacetat-Copolymer | 5,5 |
| (Luviskol^{R} VA 551) | |
| Aminomethylpropanol | 0,4 |
| PEG-60 Hydriertes Ricinusöl | 0,4 |
| Konservierungsmittel | 0,3 |
| Wasser ad | 100,0 |

aufgebracht. Nach dem Einmassieren in das Haar wurde kein Thiogeruch mehr festgestellt.
Ersatz des parfumbeladenen Polyamidpulvers durch 0,3 Gew.-% des identischen freien Parfum resultierte in einer signifikanten Geruchsverschlechterung.

### Beispiel 5

Auf entsprechend dem Verfahren nach Beispiel 1 dauergewelltes und fixiertes Haar wurde eine **"Leave-on Kur"** folgender Zusammensetzung aufgebracht:

| | |
|---|---|
| Cetylstearylalkohol | 2,5 (Gew.-%) |
| Isopropylmyristat | 0,2 |
| Stearamidopropyldimethylamin | 0,6 |
| Milchsäure | 0,3 |
| Konservierungsmittel | 0,3 |
| Mit 20 Gew.-% Parfum beladenes Polyamid- | 2,0 |
| pulver Nylon^{R} -12(mittl. Teilchendurchmesser: | |
| > 90% zwischen 3,5 und 12,5 µm) | |
| Wasser ad | 100,0 |

Das Haar zeigte keinerlei unangenehmen Geruch.

Ersatz des parfumbeladenen Polyamidpulvers durch 0,4 Gew.-% des identischen Parfums führte zu einem Haar mit typischem "Thio"-Geruch.

### Beispiel 6

Eine **Haarspülung** der folgenden Zusammensetzung:

| | |
|---|---|
| Tocopherylacetat | 0,1 (Gew.-%) |
| Cetylstearylalkohol | 3,0 |
| Mit 20 Gew.-% Parfum beladenes Polyamidpulver (ORGASOL^{R} 2002; mittl. Teilchengröße: > 90% zwischen 5 und 10 µm) | 2,5 |
| Steartrimoniumchlorid | 0,8 |
| Behenyltrimoniumchlorid | 1,2 |
| Isopropylmyristat | 1,0 |
| Paraffinöl | 1,0 |
| Hydroxypropyl Guar | 0,4 |
| Hydroxypropyltrimoniumchlorid | |
| Konservierungsmittel | 0,3 |
| Citronensäure | 0,1 |
| Wasser ad | 100,0 |

ergab nach dem Einmassieren in entsprechend dem Well- und Fixierverfahren nach Beispiel 1 frisch dauergewelltes Haar nicht nur eine exzellente haarpflegende Wirksamkeit, sondern auch einen angenehmen Geruch.
Die Behandlung mit einem Mittel, das anstelle des parfumbeladenen Polyamidpulvers 0,5 Gew.-% des gleichen Parfumöls enthielt, konnte den typischen "Dauerwell"-Geruch hingegen nicht überdecken.

### Beispiel 7

Mit einer **"Rinse off"-Kur** der Zusammensetzung

| | |
|---|---|
| Cetrimoniumchlorid | 1,5 (Gew.-%) |
| Cetylstearylalkohol | 7,0 |
| Quaternium-18 | 3,5 |
| Lanolin | 1,0 |
| Glycerin | 5,0 |
| Citronensäure | 0,1 |
| Konservierungsmittel | 0,3 |
| Mit 15 Gew.-% Parfum beladenesPolyamidpulver (Orgasol^{R}2002; durchschnittl. Teilchendurchmesser 5 bis 15µm) | 3,0 |
| Wasser ad | 100,0 |

wurde nach zehnminütiger Einwirkung auf entsprechend Beispiel 1 dauergewelltes und fixiertes Haar und anschließendem Ausspülen und Trocknen nicht nur eine Verbesserung der Haareigenschaften, sondern auch ein Verschwinden des typischen "Thio"-Geruchs erzielt.

Diese geruchsverbessernde Wirkung blieb bei der Verwendung eines identischen Mittels, das 0,45 Gew.-% des identischen freien Parfums enthielt, aus.

## Patentansprüche

1. Verfahren zum Dauerwellen von menschlichen Haaren, wobei in an sich bekannter Weise das Haar durch eine wäßrige Reduktionsmittelzusammsetzung verformt und, nach dem Ausspülen und gegebenenfalls einer Zwischenbehandlung, mit einer Oxidationsmittelzusammensetzung fixiert wird, **dadurch gekennzeichnet, daß** nach der Entfernung der Reduktionsmittelzusammensetzung und/oder der Fixiermittelzusammensetzung das Haar mit einem Präparat behandelt wird, das 1 bis 35 Gew.-% Parfum an beladenen Teilchen beträgt Parfum beladene Polyamidteilchen, von denen mindestens 90 Gew.-% eine Teilchengröße von 0,5 bis 50 Mikron aufweisen, enthält wobei die Menge an Parfum, mit denen die Teilchen beladen sind.

2. Verfahren nach Anspruch 1, **dadurch gekennzeichnet, daß** die Teilchengröße der beladenen Polyamidteilchen 1 bis 25 Mikron beträgt.

3. Verfahren nach Anspruch 1 oder 2, **dadurch gekennzeichnet, daß** das Nachbehandlungspräparat nach der Fixierung aufgebracht und nach kurzer Einwirkung wieder aus dem Haar entfernt wird.

4. Verfahren nach Anspruch 1 und/oder 2, **dadurch gekennzeichnet, daß** das Nachbehandlungspräparat nach der Fixierung aufgebracht wird und auf dem Haar verbleibt.

## Claims

1. Process for the permanent waving of human hair, wherein the hair is permanently waved in a manner known **per se** with an aqueous reducing agent composition, then, after rinsing and optionally an intermediate treatment, neutralized with an oxidizing agent composition, **characterized in that** after removing the reducing agent and/or the neutralizing composition the hair treated with a preparation containing polyamide particles charged with perfume, whereof at least 90 % by weight, have a particle size of 0.5 to 50 microns, whereby the amount of perfume with which the particles are charged is 1 to 35% by wt. perfume compared with the charged particle.

2. Process according to claim 1, wherein the particle size of the charged polyamide particles is from 1 to 25 microns.

3. Process according to claim 1 or 2, wherein the post-treatment composition is applied after neutralization and removed from the hair after short processing.

4. Process according to claim 1 and/or 2, wherein the post-treatment preparation is applied onto the hair after neutralization and remains thereon.

## Revendications

1. Procédé de déformation permanente des cheveux humains, dans lequel les cheveux sont mis en forme de manière connue en soi avec une composition aqueuse d'agent réducteur et, après un rinçage et un éventuel traitement intermédiaire, sont fixés à l'aide d'une composition d'agent oxydant, **caractérisé en ce qu'**après avoir enlevé la composition d'agent réducteur et/ou la composition d'agent fixant, les cheveux sont traités avec une préparation qui contient des particules de polyamide chargées en parfum, dont au moins 90 % en poids ont une taille comprise allant de 0,5 à 50 micromètres, la quantité de parfum dont les particules sont chargées représentant de 1 à 35 % en poids de parfum par rapport aux particules chargées.

2. Procédé selon la revendication 1, **caractérisé en ce que** la taille des particules de polyamide chargées est comprise entre 1 et 25 micromètres.

3. Procédé selon les revendications 1 ou 2, **caractérisé en ce que** la préparation de traitement complémentaire est appliquée après la fixation et est enlevée des cheveux après une action de courte durée.

4. Procédé selon les revendications 1 et/ou 2, **caractérisé en ce que** la préparation de traitement complémentaire est appliquée après la fixation et reste sur les cheveux.
